# EUROPEAN PATENT APPLICATION

(11) **EP 4 218 635 A1**
(43) Date of publication of application: **02.08.2023**
(21) Application number: 23163040.1
(22) Date of filing: 20.04.2017
(51) Int. Cl.: A61B 17/70, A61B 17/86

(54) **SYSTEMS AND METHODS FOR A PEDICLE SCREW ASSEMBLY**

(30) Priority: 20.04.2016 US 201662324972 P
(62) Divisional of application: 17786654.8
(71) Applicant: Dignity Health, San Francisco, CA 94107 (US)
(72) Inventor: BOHL,, Michael Austin, Phoenix, 85028 (US)
(74) Representative: Jones, Nicholas Andrew

(57) **Abstract**

Various embodiments of a pedicle screw assembly having a pedicle screw defining an axial channel in communication with a pair of lateral channels configured to receive a bone anchor therein that extends outwardly from the pair of lateral channels in a post-deployment position for anchoring the pedicle screw to bone tissue are disclosed.

## Description

### FIELD

The present disclosure generally relates to surgical devices and in particular to systems and methods for pedicle screw assemblies.

### BACKGROUND

Mechanical instability of the spine can result from many causes, including degenerative disease, trauma, infection, spinal deformity, or neoplastic processes. Left untreated, such spinal instability can result in pain, neurological compromise, and immobility. Spinal instability is treated surgically via fixation and fusion of the unstable spinal levels. Fixation of the spine is accomplished posteriorly through the insertion of screws through the vertebral pedicles, which are then segmentally connected by metal rods.

For patients undergoing spinal fixation and fusion surgery, pedicle screws are considered a standard of care. One of the most common complications associated with pedicle screw fixation, especially in long fixation constructs, is screw failure and pull-out with resultant pseudarthrosis or adjacent segment disease. Existing strategies for increasing the axial resistance required to pull out a pedicle screw include increased screw sizes, alternative core shapes, alternative thread shapes, and bone-cement augmentation. Each of these strategies comes with significant limitations, and none have proven to be a clinically reliable solution to the problem of pedicle screw failure.

It is with these observations in mind, among others, that various aspects of the present disclosure were conceived and developed.

### BRIEF DESCRIPTION OF THE DRAWINGS

FIG. **1** is a perspective view of a first embodiment of a pedicle screw assembly having a pedicle screw and a bone anchor, according to one aspect of the present disclosure;
FIG. **2** is an exploded view of the pedicle screw assembly of FIG. 1 depicting the pedicle screw and the bone anchor, according to one aspect of the present disclosure;
FIG. **3** is a cross-sectional view of the pedicle screw taken along line **3-3** of FIG. **2****,** according to one aspect of the present disclosure;
FIG. **4** is a cross-sectional view of the pedicle screw of FIG. **3** showing the bone anchor disposed partially within a channel of the pedicle screw, according to one aspect of the present disclosure;
FIG. **5** is a cross-sectional view of the pedicle screw of FIG. **3** showing the bone anchor disposed fully within the channel of the pedicle screw and deployed through an opening formed at the distal end of the pedicle screw, according to one aspect of the present disclosure;
FIG. **6** is an anatomical perspective view of the pedicle screw partially engaged inside a pedicle vertebra with the bone anchor in a pre-deployment position, according to one aspect of the present disclosure;
FIG. **7** is an anatomical perspective view of the pedicle screw partially engaged inside a pedicle vertebra with the anchor in a post-deployment position, according to one aspect of the present disclosure;
FIG. **8** is a perspective view of a second embodiment of a pedicle screw assembly showing a pedicle screw having a pair of bone anchors deployed from a pedicle screw by a driveshaft, according to one aspect of the present disclosure;
FIG. **9** is a perspective view of the pedicle screw of FIG. **8****,** according to one aspect of the present disclosure;
FIG. **10** is a cross-sectional view of the pedicle screw taken along line **10-10** of FIG. **9****,** according to one aspect of the present disclosure;
FIG. **11** is an exploded view of the pedicle screw assembly of FIG. **8** showing the pedicle screw, driveshaft and pair of bone anchors, according to aspects of the present disclosure;
FIG. **12** is a perspective view of the driveshaft, according to one aspect of the present disclosure
FIG. **13** is an end view of the driveshaft showing the spline, according to one aspect of the present disclosure;
FIG. **14** is a side view of the driveshaft, according to one aspect of the present disclosure;
FIG. **15** is a perspective view of one of the bone anchors, according to one aspect of the present disclosure;
FIG. **16** is a side view of the bone anchor of FIG. **15** showing a gear teeth arrangement, according to one aspect of the present disclosure;
FIG. **17** is an opposite side view of the bone anchor of FIG. **16****,** according to one aspect of the present disclosure;
FIG. **18** is a cross-sectional view of the pedicle screw assembly showing the pair of bone anchors in a pre-deployment position, according to one aspect of the present disclosure;
FIG. **19** is a cross-sectional view of the pedicle screw assembly showing the pair of bone anchors in a post-deployment position, according to one aspect of the present disclosure;
FIG. **20** is a top view of the pedicle screw assembly of FIG. **19** showing the pair of bone anchors in the post-deployment position, according to one aspect of the present disclosure;
FIG. **21A** is a cross-sectional view of the pedicle screw assembly showing the pair of bone anchors in the pre-deployment position shown in FIG. **18****;** and FIG. **21B** is a cross-sectional view of the pedicle screw assembly showing the pair of bone anchors in the post-deployment position when the driveshaft is actuated, according to one aspect of the present disclosure;
FIG. **22** is an anatomical perspective view of the pedicle screw partially engaged inside a pedicle vertebra with the pair of anchors in the pre-deployment position, according to one aspect of the present disclosure;
FIG. **23** is an anatomical perspective view of the pedicle screw partially engaged inside a pedicle vertebra with the pair of bone anchors in the post-deployment position, according to one aspect of the present disclosure;
FIG. **24** is a perspective view of a third embodiment of the pedicle screw assembly showing a pedicle screw and bone anchor, according to one aspect of the present disclosure;
FIG. **25** is an exploded view of the pedicle screw assembly of FIG. **24** showing the pedicle screw and anchor, according to one aspect of the present disclosure;
FIG. **26** is a cross-sectional view of the pedicle screw taken along line **26-26** of FIG. **25****,** according to one aspect of the present disclosure;
FIG. **27** is a cross-sectional view of the pedicle screw showing the bone anchor in the pre-deployment position, according to one aspect of the present disclosure;
FIG. **28** is a cross-sectional view of the pedicle screw showing the bone anchor in the post-deployment position, according to one aspect of the present disclosure;
FIG. **29** is an anatomical perspective view of the pedicle screw partially engaged inside the pedicle vertebra with the anchor shown in the pre-deployment position, according to one aspect of the present disclosure;
FIG. **30** is an anatomical perspective view of the pedicle screw partially engaged inside the pedicle vertebra with the bone anchor shown in the post-deployment position, according to one aspect of the present disclosure;
FIG. **31** is a perspective view of a fourth embodiment of the pedicle screw assembly showing a pedicle screw and bone anchor, according to one aspect of the present disclosure;
FIG. **32** is an exploded view of the pedicle screw assembly of FIG. **31** showing the pedicle screw and bone anchor, according to one aspect of the present disclosure;
FIG. **33** is a cross-sectional view of the pedicle screw assembly taken along line **33-33** of FIG. **32****,** according to one aspect of the present disclosure;
FIG. **34** is a cross-sectional view of the pedicle screw assembly showing the bone anchor in a pre-deployment position, according to one aspect of the present disclosure;
FIG. **35** is a cross-sectional view of the pedicle screw showing the bone anchor in the post-deployment position, according to one aspect of the present disclosure;
FIG. **36** is an anatomical perspective view of the pedicle screw partially engaged inside the pedicle vertebra with the bone anchor shown in the pre-deployment position, according to one aspect of the present disclosure; and
FIG. **37** is an anatomical perspective view of the pedicle screw partially engaged inside the pedicle vertebra with the bone anchor shown in the post-deployment position, according to one aspect of the present disclosure.

Corresponding reference characters indicate corresponding elements among the view of the drawings. The headings used in the figures do not limit the scope of the claims.

### DETAILED DESCRIPTION

Various embodiments of a pedicle screw assembly having one or more bone anchors that are actuated for securing a pedicle screw within a pedicle of a vertebra are disclosed. In some embodiments, the pedicle screw assembly includes a pedicle screw having external threads configured to allow the pedicle screw to be partially engaged within a pedicle vertebra. In addition, various embodiments of the pedicle screw may include a pair of opposite distal openings configured to allow passage of a bone anchor through an axial channel of the pedicle screw. This allows the bone anchor to extend outwardly from the pedicle screw when the bone anchor is in a post-deployment position to further engage the pedicle screw to the pedicle vertebra.

In some embodiments, the bone anchor includes an elongated body having a bendable portion that allows a distal portion of the bone anchor to be oriented at an angle relative to the longitudinal axis of the pedicle screw when extending outwardly from one of the distal openings. In some embodiments, the bone anchor includes an elongated body having a distal portion that defines a first lateral extension and a second lateral extension that are biased to be oriented at an acute angle relative to the longitudinal axis of the pedicle screw when the bone anchor extends outwardly from the distal openings of the pedicle screw in a post-deployment position. In some embodiments, the bone anchor includes an elongated body having a distal portion that defines a first lateral extension and a second lateral extension that are biased to be oriented at an obtuse angle relative to the longitudinal axis of the pedicle screw when the bone anchor extends outwardly from the distal openings of the pedicle screw in the post-deployment position. In some embodiments, the bone anchor may be a first bone anchor and a second bone anchor each having gear teeth configured to engage a spline portion of a driveshaft such that rotation of the driveshaft causes the first and second bone anchors to extend laterally outward from the distal openings of the pedicle screw in a post-deployment position.

In some embodiments, the pair of opposite distal openings of the pedicle screw may communicate with respective first and second lateral channels that are oriented at an obtuse angle relative to the longitudinal axis of the pedicle screw. In some embodiments, the pair of opposite distal openings of the pedicle screw may communicate with respective first and second lateral channels that are oriented at an acute angle relative to the longitudinal axis of the pedicle screw. In some embodiments, the pair of opposite distal openings of the pedicle screw may communicate with respective first and second lateral channels that are oriented at a perpendicular angle relative to the longitudinal axis of the pedicle screw.

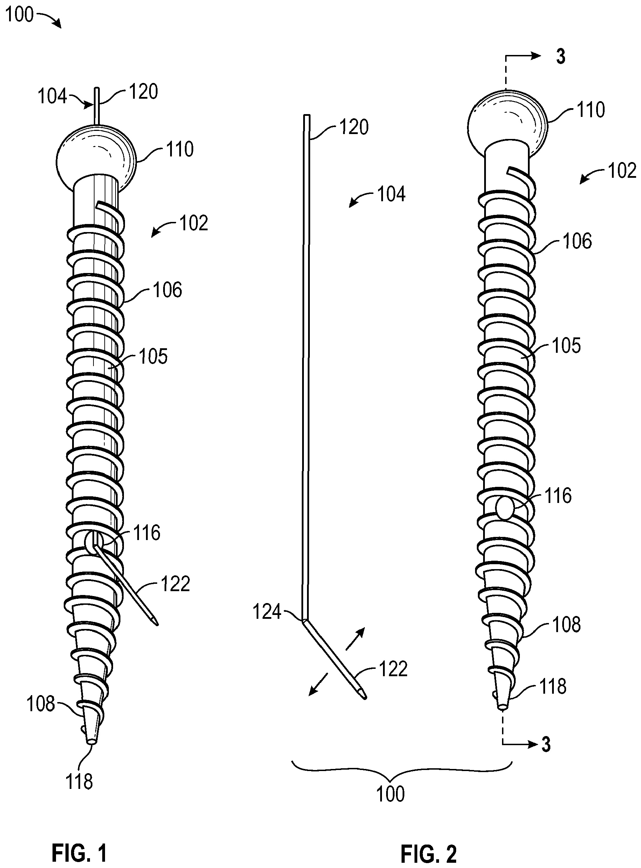

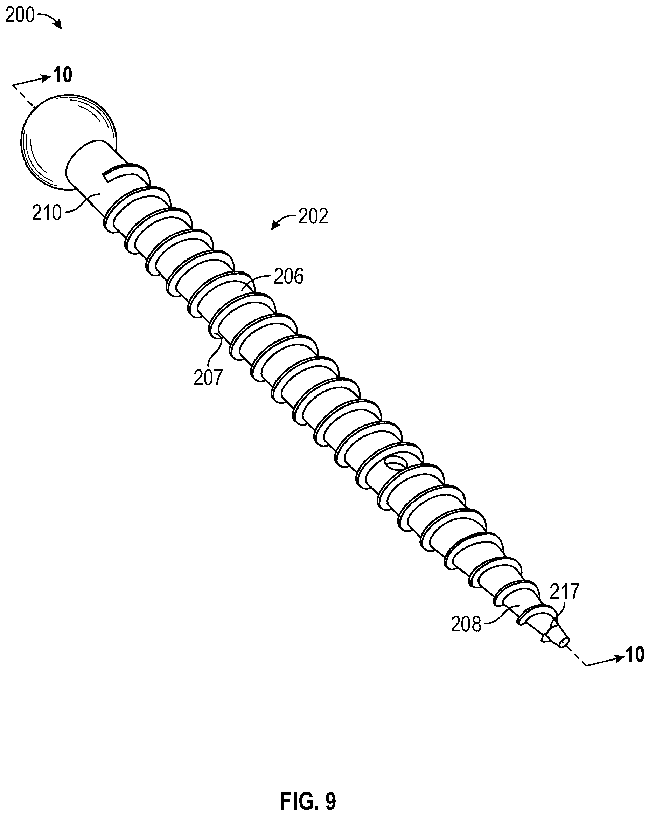

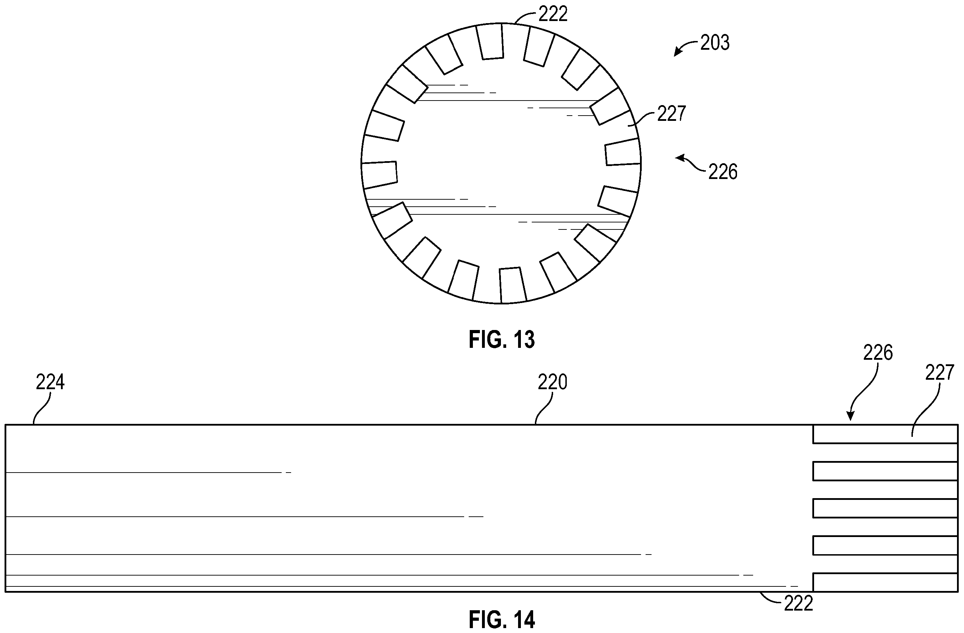

In one method of engaging a pedicle screw to a bone portion, a pedicle screw is inserted into a pedicle of the vertebra and a bone anchor is inserted through the pedicle screw such that the distal portion of the bone anchor extends outwardly from the pedicle screw in a post-deployment position to further engage the pedicle screw to the pedicle vertebra. In another method of engaging a pedicle screw to a bone portion, a pedicle screw is inserted into a pedicle of the vertebra and a pair of bone anchors disposed within one or more respective lateral channels in a pre-deployment position are caused to extend outwardly to a post-deployment position from the pedicle screw upon actuation of a driveshaft that is operatively engaged to each bone anchor to further engage the pedicle screw to the pedicle vertebra. Referring to the drawings, embodiments of a pedicle screw assembly are illustrated and generally indicated as **100, 200, 300** and **400** in FIGS. **1-37****.**

Referring to FIGS. **1-7****,** a first embodiment of a pedicle screw assembly, designated **100,** is illustrated. As shown in FIGS. **1-3****,** the pedicle screw assembly **100** includes a pedicle screw **102** that is configured to be coupled to a bone anchor **104** for engaging a portion of tissue, such as the bone tissue of the vertebra. In some embodiments, the pedicle screw **102** defines an elongated body **105** forming a distal portion **108** defining a conical tip **118** at the free end thereof and a proximal portion **110** that defines a proximal opening **114** in communication with an axial channel **112** formed along the longitudinal axis of the pedicle screw **102.** In some embodiments, the elongated body **105** defines a threaded portion **106** that extends substantially along the length of the elongated body **105** configured to permit the pedicle screw **102** to engage and retained within the bone tissue.

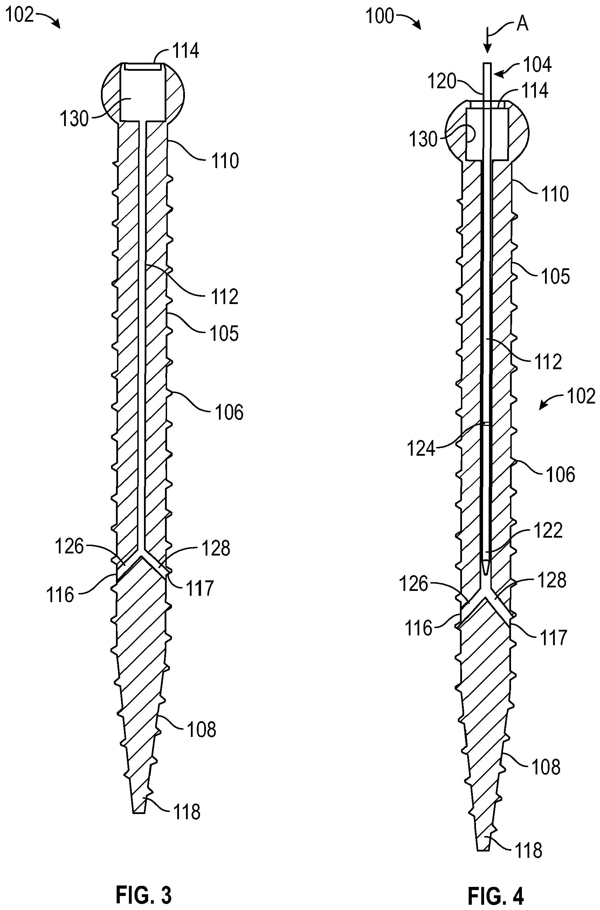

Referring to FIG. **3****,** proximal opening **114** communicates with the one end of the axial channel **112** through a cavity **130** formed proximate the proximal opening **114,** while the opposite end of the axial channel **112** communicates with a pair of first and second lateral channels **116** and **117** that extend at an obtuse angle (downward angle) relative to the axial channel **112.** As shown, the first lateral channel **126** communicates with a first lateral opening **116** and the second lateral channel **128** communicates with a second lateral opening **117** which are both formed along the distal portion **108** of the pedicle screw **102.**

Referring back to FIG. **2****,** in some embodiments the bone anchor **104** defines an elongated proximal portion **120** and a distal portion **122** configured to be received through the axial channel **112** and one of either the first or second lateral channels **126** and **128** such that the distal portion **122** of the bone anchor **104** extends outwardly from either the first or second lateral openings **116** and **117** in a post-deployment position. In some embodiments, the bone anchor **104** defines a bendable portion **124** formed between the proximal portion **120** and distal portion **122** of the bone anchor **104** that allows the distal portion **122** of the bone anchor **104** to bend or hinge relative to the proximal portion **120** as the bone anchor **104** extends through either the first or second lateral openings **116** and **117.**

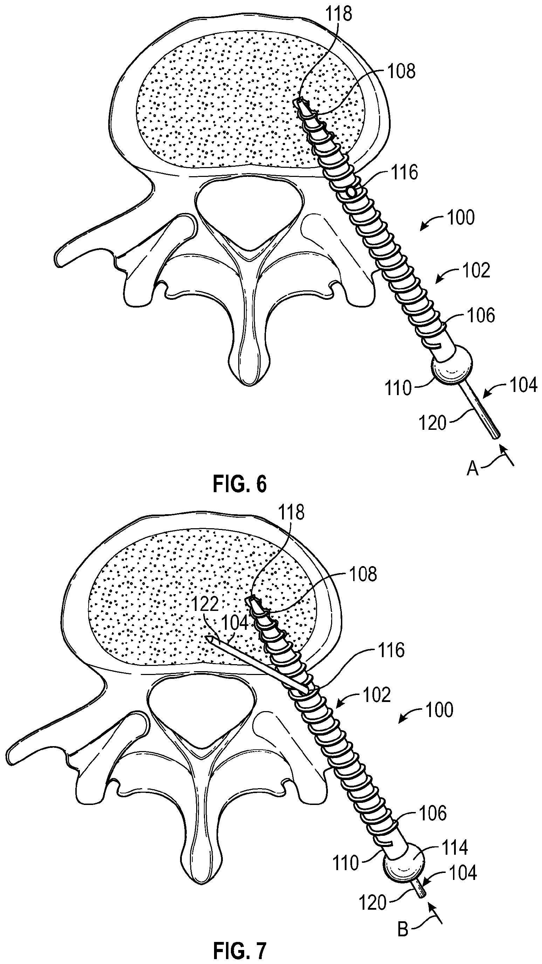

Referring to FIGS. **4-7****,** one method of implanting the pedicle screw assembly **100** will be discussed. As shown in FIG. **6****,** the pedicle screw **102** is first engaged into the bone tissue, for example a pedicle of the vertebra, such that the proximal portion **110** of the pedicle screw **102** extends from the bone tissue. Once the pedicle screw **102** is implanted into the bone tissue, the bone anchor **104** is inserted into the proximal opening **114** of the pedicle screw **102** along direction **A** until disposed within the axial channel **112** proximate the first and second lateral channels **126** and **128** in a pre-deployment position as illustrated in FIG. **4****.**

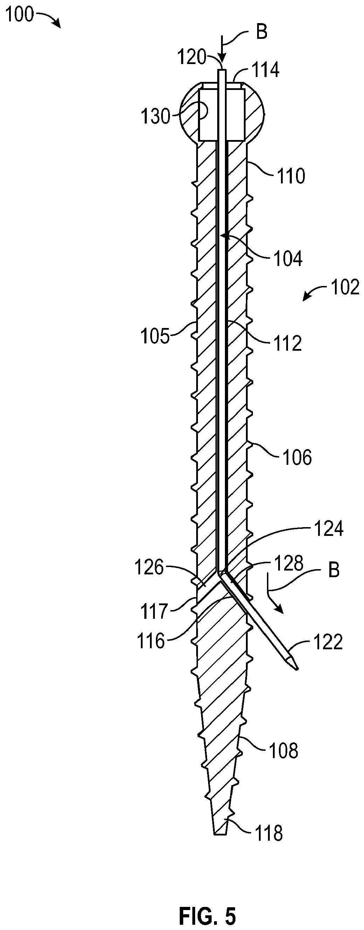

Once in the pre-deployment position, in some embodiments the user may then further insert the bone anchor **104** in direction **B** until the distal portion **122** of the bone anchor **104** travels through either the first or second lateral channels **126** and **128** until extending outwardly from either the first or second lateral openings **116** and **117** in a post-deployment position as illustrated in FIG. **5****.** In this post-deployment position, the distal portion **122** of the bone anchor is engaged to the bone tissue as shown in FIG. **7****.**

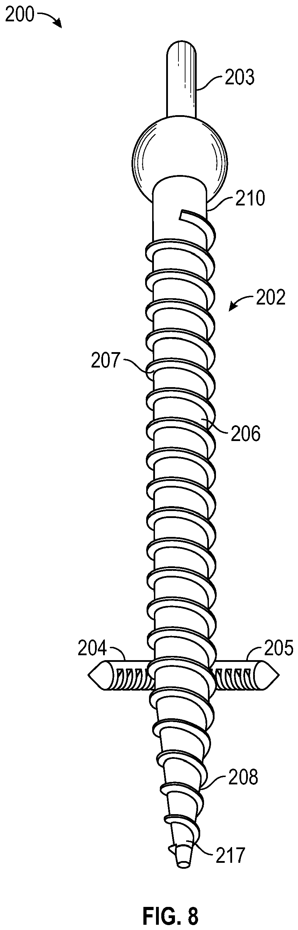

Referring to FIGS. **8-23****,** a second embodiment of the pedicle screw assembly, designated **200,** is illustrated. As shown in FIGS. **8-11****,** the pedicle screw assembly **200** includes a pedicle screw **202** that is configured to be coupled to a driveshaft **203** which is in operative engagement with a pair of first and second bone anchors **204** and **205** for anchoring the pedicle screw **202** into bone tissue, such as a pedicle vertebra. In some embodiments, the pedicle screw **202** defines an elongated body **206** forming a distal portion **208** defining a conical tip **217** at the free end thereof and a proximal portion **210** that defines a proximal opening **216** in communication with an axial channel **212** formed along the longitudinal axis of the pedicle screw **202.** In some embodiments, the elongated body **206** defines a threaded portion **207** that extends substantially along the length of the elongated body **206** configured to permit the pedicle screw **202** to engage and be retained within the bone tissue.

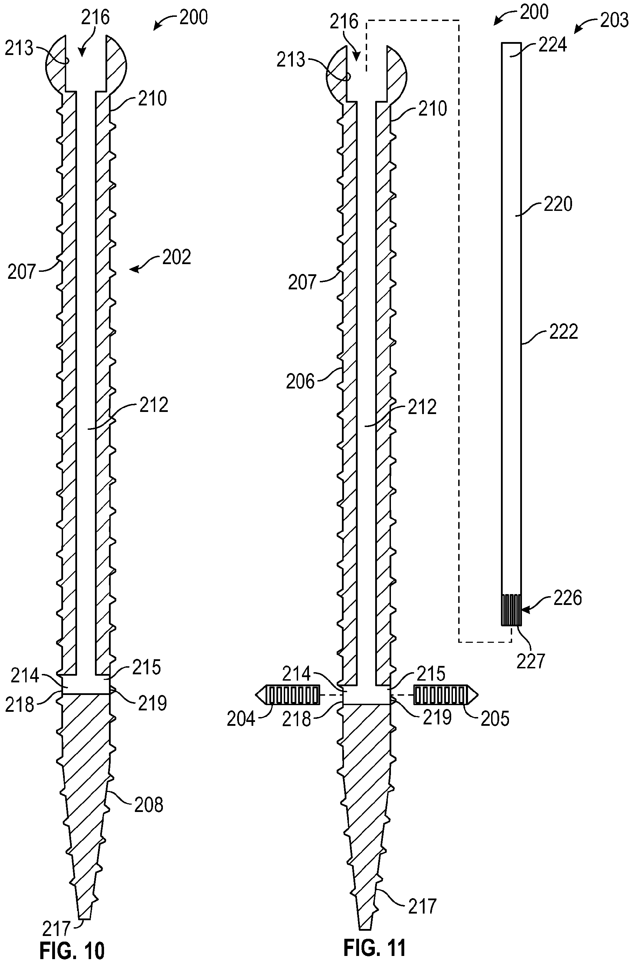

Referring to FIGS. **10** and **11****,** proximal opening **216** communicates with the one end of the axial channel **212** through a cavity **213** formed proximate the proximal opening **216,** while the opposite end of the axial channel **212** communicates with a pair of first and second lateral channels **214** and **215** that extend at a substantially perpendicular angle relative to the longitudinal axis of the axial channel **212.** As shown, the first lateral channel **214** communicates with a first lateral opening **218** and the second lateral channel **215** communicates with a second lateral opening **219** which are both formed generally adjacent to the distal portion **208** of the pedicle screw **202.**

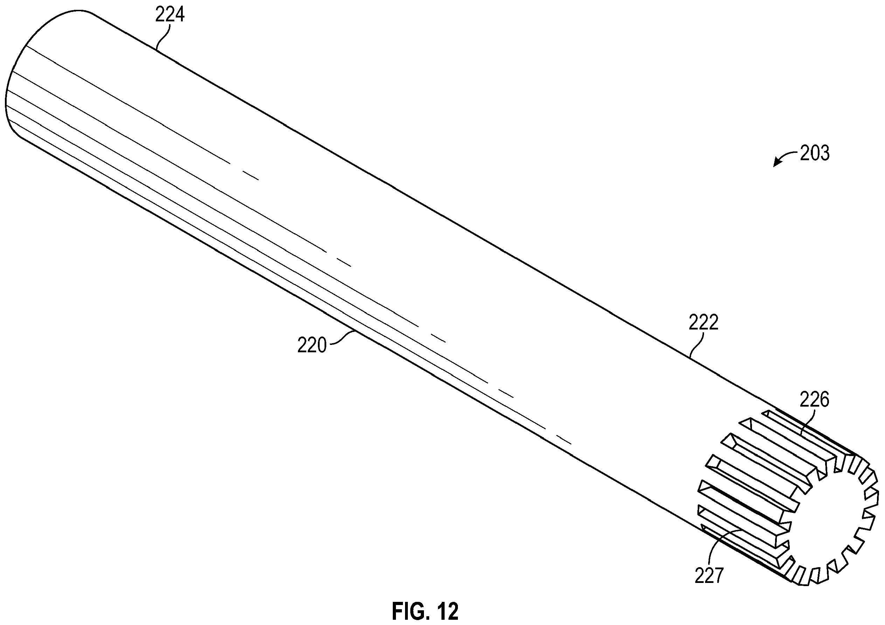

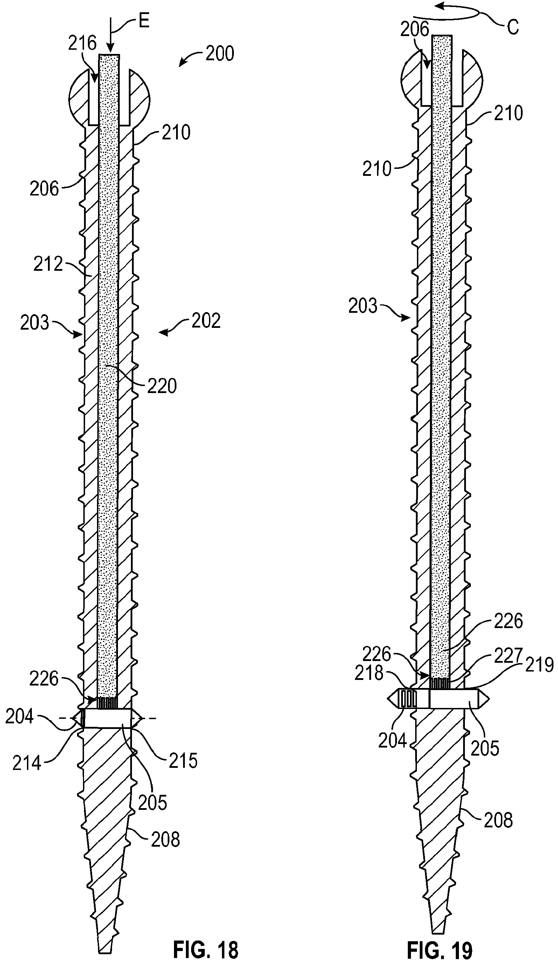

Referring specifically to FIGS. **12-14****,** **18** and **19****,** the driveshaft **203** is operative to actuate the first and second bone anchors **204** and **205** from a pre-deployment position (FIG. **18**) with the first and second bone anchors **204** and **205** fully disposed within the first and second lateral channels **214** and **215,** respectively, and a post-deployment position (FIG. **19**) with the first and second bone anchors **204** and **205** extending outwardly from the first and second channels **214** and **215,** respectively. In operation, rotation of the driveshaft **203** causes the first and second bone anchors **204** and **205** to extend laterally to the post-deployment position from the first and second lateral openings **218** and **219,** respectively, as shall be discussed in greater detail below.

Referring to FIGS. **12-14****,** in some embodiments the driveshaft **203** defines an elongated body **220** defining a distal portion **222** and a proximal portion **224.** As shown, the distal portion **222** of the drive shaft **203** defines a spline **226** that forms a plurality of axially extending peripheral slots **227** formed along the periphery of the spline **226.** The spline **226** is configured to operatively engage at least one of the first and second bone anchors **204** and **205** as discussed below.

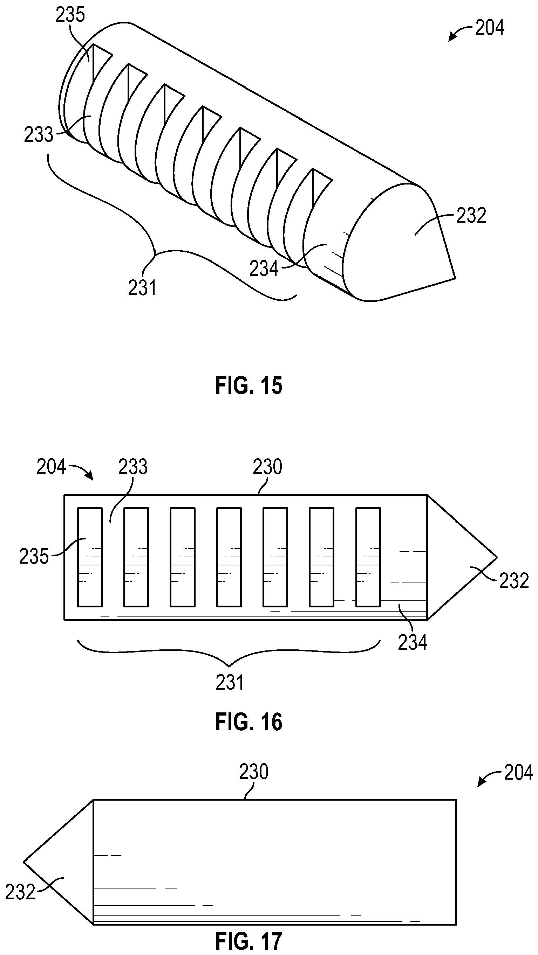

In some embodiments, the first and second bone anchors **204** and **205** have identical shapes and are configured to be disposed within the first and second lateral channels **214** and **215,** respectively, of the pedicle screw **202** when in the pre-deployment position shown in FIG. **18****.** By way of example, the first bone anchor **204,** which is identical to the second bone anchor **205,** will be described in reference to FIGS. **15-17** with the description being applicable to both first and second bone anchors **204** and **205.** In some embodiments, each bone anchor **204** and **205** has an anchor body **230** defining a pointed end **232** configured to penetrate bone tissue when the first and second bone anchors **204** and **205** are in the post-deployment position as shown in FIG. **8****.** In addition, the anchor body **230** has a lateral side **234** forming a gear teeth arrangement **231** configured to engage the spline **226** of the driveshaft **203.** In some embodiments, the gear teeth arrangement **231** defines a plurality of ridges **233** and slots **235** in which each ridge **233** is formed between a respective pair of slots **235** along a length of the gear teeth arrangement **231.** In one method of engagement between the first and second bone anchors **204** and **205** and the driveshaft **203,** a respective portion of the spline **226** for the driveshaft **203** engages the respective gear teeth arrangement **233** of the first and second bone anchors **204** and **205** such that rotation of the driveshaft **203** advances the first and second bone anchors **204** and **205** outwardly from the pedicle screw **202.**

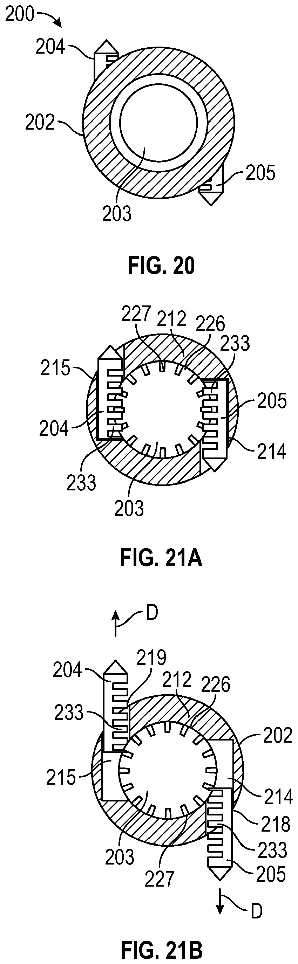

As noted above, the driveshaft **203** is configured to be disposed within the axial channel **212** such that the spline **226** operatively engages the first and second bone anchors **204** and **205** disposed within the first and second lateral channels **214** and **215,** respectively, when the pedicle screw assembly **100** is in the pre-deployment position as shown in FIG. **18****.** In the pre-deployment position shown in the cross-sectional views of FIGS. **18** and **21A****,** the first and second anchors **204** and **205** are disposed within the first and second lateral channels **214** and **215** such that the gear teeth arrangement **233** of the first and second bone anchors **204** and **205** are operatively engaged to the spline **226** of the driveshaft **203.** In the post-deployment position shown in the cross-sectional views of FIGS. **19** and **21B****,** rotation of the driveshaft **203** in a counter-clockwise direction causes the spline **226** to continuously engage respective gear teeth arrangement **233** such that the first and second bone anchors **204** and **205** are incrementally moved outwardly from the first and second lateral channels **214** and **215** as illustrated in FIG. **20****.** In other embodiments, the driveshaft **203** and the first and second bone anchors **204** and **205** may be operatively engaged such that rotation of the driveshaft **203** in the opposite clockwise direction also incrementally moves the first and second bone anchors **204** and **205** outwardly from the first and second lateral channels **214** and **215.**

Referring back to FIG. **21A****,** in some embodiments the first and second lateral channels **214** and **215** are formed in an off-set arrangement on either side of the axial channel **212** and are in communication thereto. As such, the gear teeth arrangement **233** of the first and second bone anchors **204** and **205** are in operative engagement with the spline **226** when the driveshaft **203** is fully disposed within the axial channel **212.**

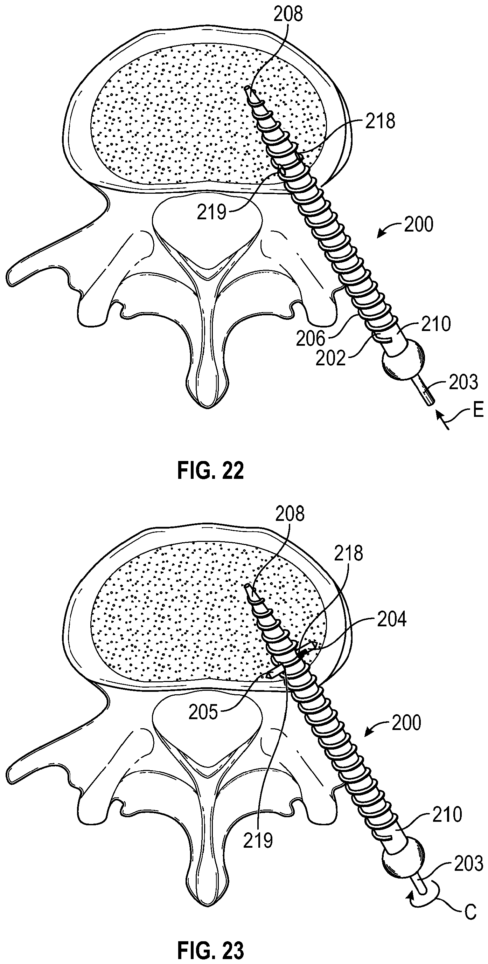

Referring to FIGS. **22** and **23****,** one method of implanting the pedicle screw assembly **200** will be discussed. As shown in FIG. **22****,** the pedicle screw **202** is first engaged into the bone tissue, such as a pedicle vertebra, such that the proximal portion **210** of the pedicle screw **102** extends from the bone tissue and the distal portion **208** of the pedicle screw **202** is partially or fully implanted inside the bone tissue. Once the pedicle screw **202** is implanted into the bone tissue, the driveshaft **203** is inserted into the axial channel **212** of the pedicle screw **202** along axial direction **E** until disposed within the axial channel **212** proximate the first and second lateral channels **214** and **215.** This insertion of the driveshaft **203** into the pedicle screw **202** allows the driveshaft **203** to be in operative engagement with the first and second bone anchors **204** and **205** already disposed within the first and second lateral channels **214** and **215,** respectively, thereby placing the pedicle screw assembly **200** is in a pre-deployment position.

Once in the pre-deployment position, the user may then rotate the driveshaft **203** in the counter-clockwise direction **C** until the first and second bone anchors **204** and **205** are fully extended into the bone tissue by the rotation of the driveshaft **203.** As such, the pedicle screw assembly **200** assumes a post-deployment position as shown in FIG. **23****.**

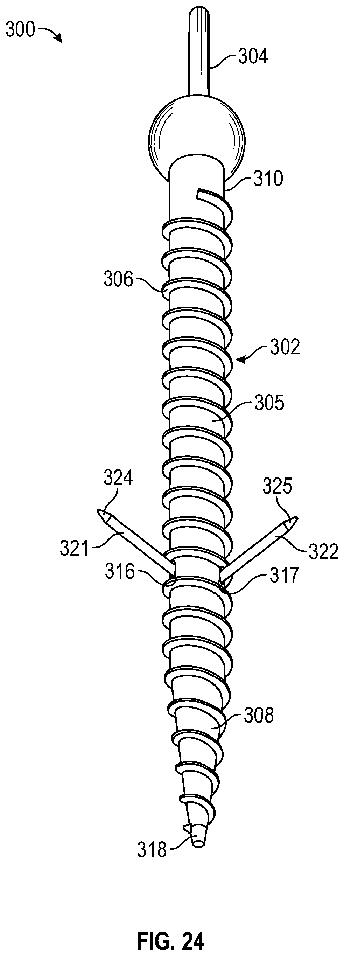

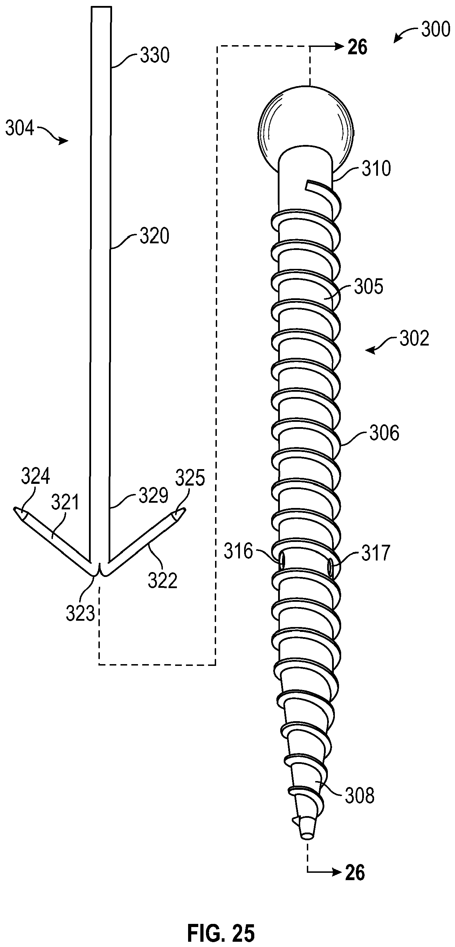

Referring to FIGS. **24-30****,** a third embodiment of a pedicle screw assembly, designated **300,** is illustrated. As shown in FIGS. **24** and **25****,** the pedicle screw assembly **300** includes a pedicle screw **302** that is configured to be coupled to a bone anchor **304** for engaging a portion of bone, such as a pedicle vertebra. In some embodiments, the pedicle screw **302** defines an elongated body **305** forming a distal portion **308** defining a conical tip **318** at the free end thereof and a proximal portion **310** that defines a proximal opening **314** in communication with an axial channel **312** formed along the longitudinal axis of the pedicle screw **302.** In some embodiments, the elongated body **305** defines a threaded portion **306** that extends substantially along the length of the elongated body **305** configured to permit the pedicle screw **302** to engage and be retained within the bone tissue.

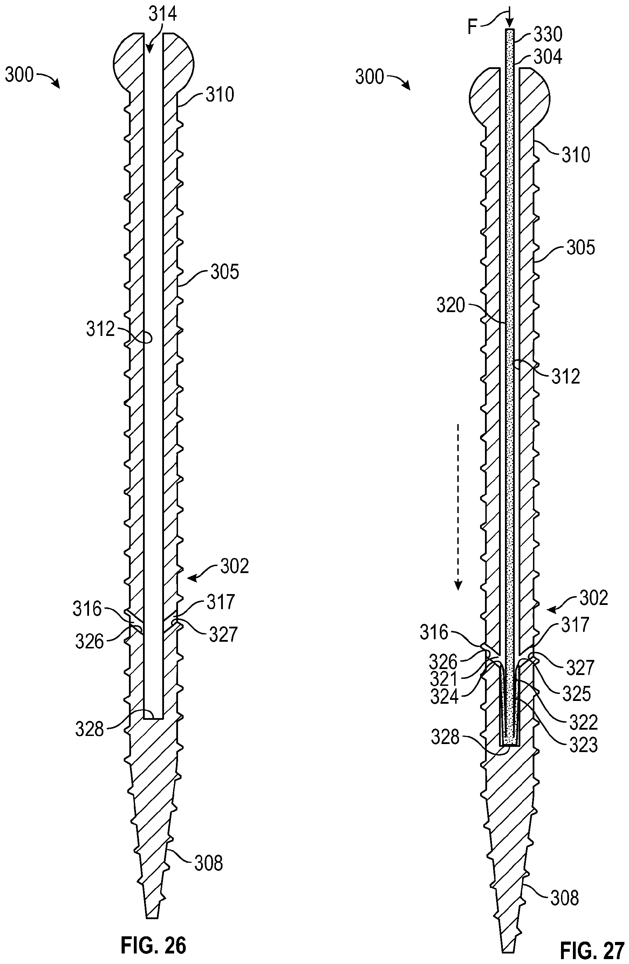

Referring to FIG. **26****,** the proximal opening **314** communicates with one end of the axial channel **312,** while the opposite end of the axial channel **312** communicates with a pair of first and second lateral channels **326** and **327** that extend at an acute (upward) angle relative to longitudinal axis of the axial channel **312.** As shown, the first lateral channel **326** communicates with a first lateral opening **316** and the second lateral channel **327** communicates with a second lateral opening **317** which are both formed along the distal portion **308** of the pedicle screw **302.**

Referring back to FIGS. **24** and **25****,** in some embodiments the bone anchor **304** defines an elongated body **320** forming an elongated proximal portion **330** and a distal portion **329** configured to be received through the axial channel **312** of the pedicle screw **302.** The bone anchor **304** further defines an apex **323** at the distal portion **329** with a first lateral extension **321** and a second lateral extension **322** extending from the apex **323** and the distal portion **329** of the bone anchor **304.** In some embodiments, the first lateral extension **321** of the bone anchor **304** includes a first pointed end **324,** and the second lateral extension **322** of the bone anchor **304** includes a second pointed end **325.** The bone anchor **304** is depicted in FIG. **25** in an unbiased state with the first lateral extension **321** and the second lateral extension **322** extending laterally away from the elongated body **320** of the bone anchor **304.**

In a pre-deployment position, the bone anchor **304** is inserted into and disposed within the axial channel **312** in direction **F** (FIG. **27**)and assumes a biased state as the first lateral extension **321** and the second lateral extension **322** are forced together toward the elongated proximal portion **330** due to the small width of the axial channel **312.** Specifically, the first lateral extension **321** and the second lateral extension **322** become temporarily biased together or otherwise bent inwardly toward each other within the axial channel **312** such that the first and second pointed ends **324** and **325** are positioned generally adjacent to the first and second lateral channels **326** and **327** as illustrated in FIG. **27** In other words, manipulating the first lateral extension **321** and the second lateral extension **322** into the pre-deployment position described above temporarily configures the bone anchor **304** in a substantially linear shape which allows the bone anchor **304** to be slidably received within the axial channel **312** of the pedicle screw **302.** The bone anchor may be urged all the way through the axial channel **312** such that the apex **323** of the bone anchor **304** makes contact with or at least substantially reaches a terminal ending **328** of the axial channel **312** of the pedicle screw **302.**

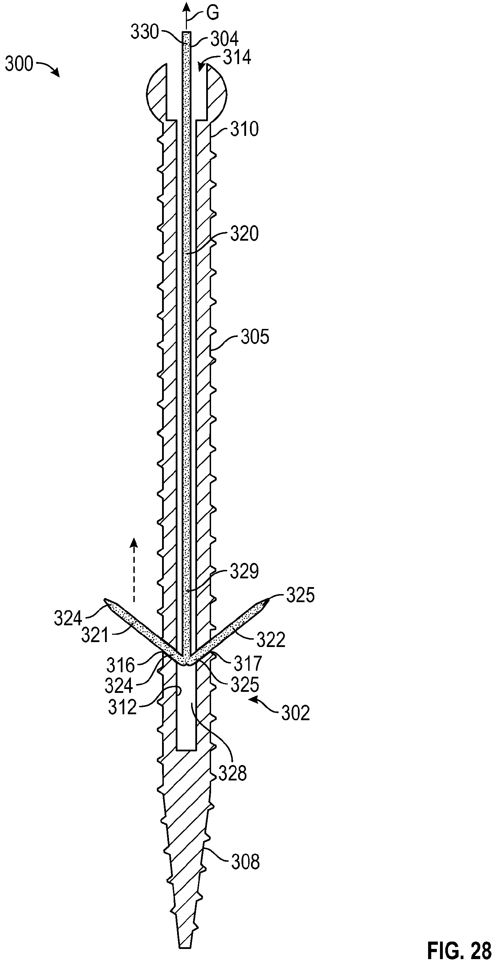

Referring to FIGS. **27-28****,** the bone anchor **304** may be manipulated from a pre-deployment position to a post-deployment position with the first lateral extension **321** and the second lateral extension **322** oriented away from the elongated body **320** and extending through the first lateral opening **316** and the second lateral opening **317,** respectively. In this post-deployment position, the first lateral extension **321** and the second lateral extension **322** generally return to an unbiased state. Specifically, to assume the post-deployment position, the bone anchor **304** is pulled in a direction **G** (FIG. **28**) opposite the direction **F** to orient the bone anchor **304** such that the first pointed end **324** and the second pointed end **325** positioned below the first lateral opening **316** and the second lateral opening **317** respectively may then spring through the first lateral channel **326** and the first lateral opening **316,** and the second lateral extension **322** may spring through the second lateral channel **327** and the second lateral opening **317** to assume the post-deployment position shown in FIG. **28****.**

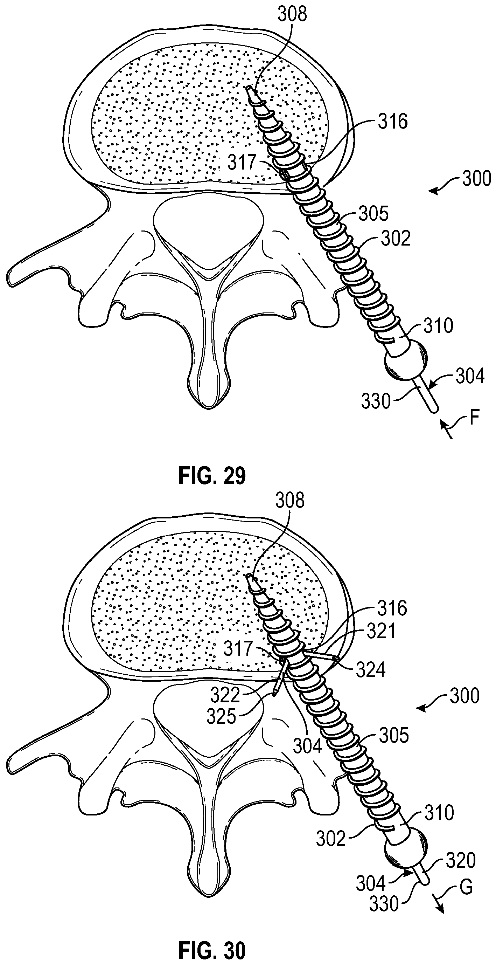

Referring to FIGS. **29-30****,** one method of implanting the pedicle screw assembly **300** will be discussed. As shown in FIG. **29****,** the pedicle screw 302 is first engaged into a bone tissue, such as a pedicle vertebra, such that the proximal portion **310** of the pedicle screw **302** extends from the bone tissue. Once the pedicle screw **302** is implanted into the bone tissue, the bone anchor **304** is inserted through the proximal opening **314** and into the axial channel **312** of the pedicle screw **302** along the direction **F** until disposed within the axial channel **312** proximate the first and second lateral channels **326** and **327.** The bone anchor **304** may then be pulled back slightly in the direction **G** opposite the direction **F** to bring the bone anchor **304** into the post-deployment position of FIG. **30****.** In the post deployment position, the first lateral extension **321** extends through the first lateral opening **316** with the first pointed end **324** extending into the bone tissue, and the second lateral extension **322** extends through the second lateral opening **317** with the second pointed end **325** extending into the bone tissue.

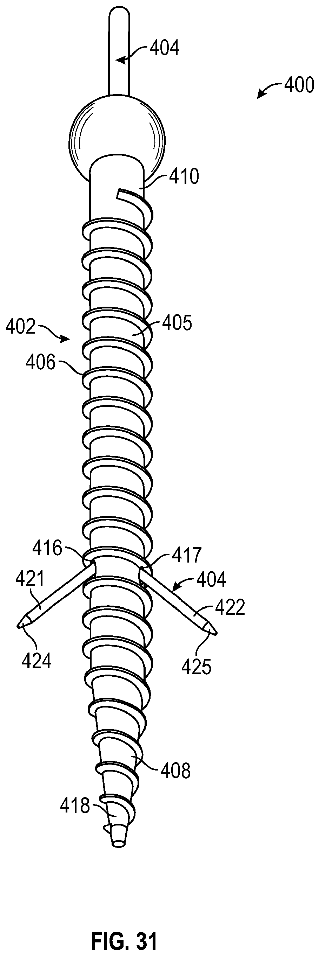

Referring to FIGS. **31-37****,** a fourth embodiment of the pedicle screw assembly, designated **400** is illustrated. As shown in FIGS. **31-33****,** the pedicle screw assembly **400** includes a pedicle screw **402** that is configured to be coupled to a bone anchor **404** for engaging a portion of bone tissue, such as a pedicle vertebra. In some embodiments, the pedicle screw **402** defines an elongated body **405** forming a distal portion **408** defining a conical tip **418** at the free end thereof and a proximal portion **410** that defines a proximal opening **414** in communication with an axial channel **412** formed along the longitudinal axis of the pedicle screw **402.** In some embodiments, the elongated body **405** defines a threaded portion **406** substantially along the length of the elongated body **405** to permit the pedicle screw **402** to be screwed into and retained within the bone tissue.

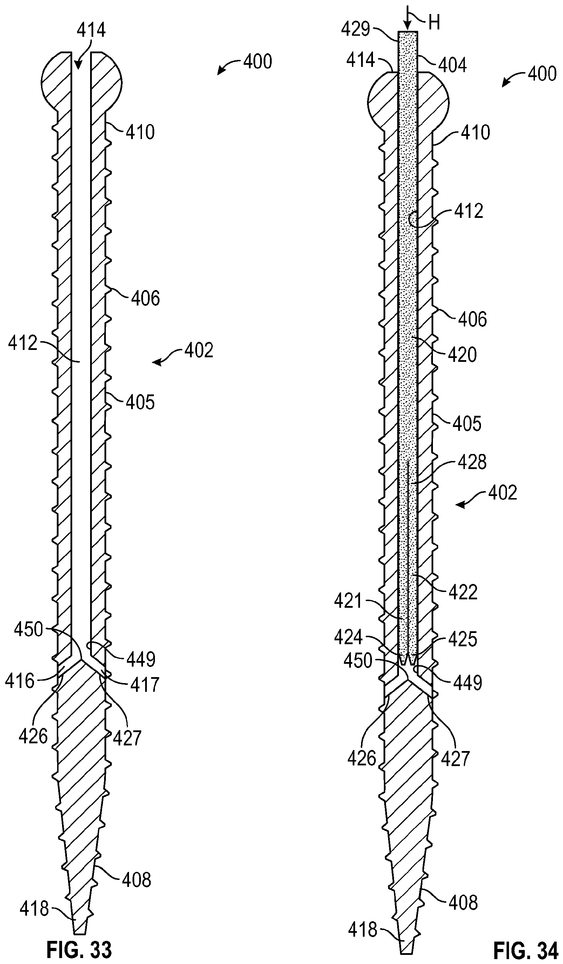

Referring to FIG. **33****,** the proximal opening **414** communicates with one end of the axial channel **412,** while the opposite end of the axial channel **412** communicates with a pair of first and second lateral channels **426** and **427** that extend at an obtuse angle (downward angle) relative to the axial channel **412.** As shown, the first lateral channel **426** communicates with a first lateral opening **416** and the second lateral channel **427** communicates with a second lateral opening **417** which are both formed along the distal portion **408** of the pedicle screw **402.** An apex **450** is formed at a terminal end **449** of the axial channel **412** and may be defined between the first and second lateral channels **426** and **427.** In some embodiments, the apex **450** is triangle-shaped to facilitate the guidance of the first lateral extension **421** through the first lateral channel **426,** and the second lateral extension **422** through the second lateral channel **427,** as described herein.

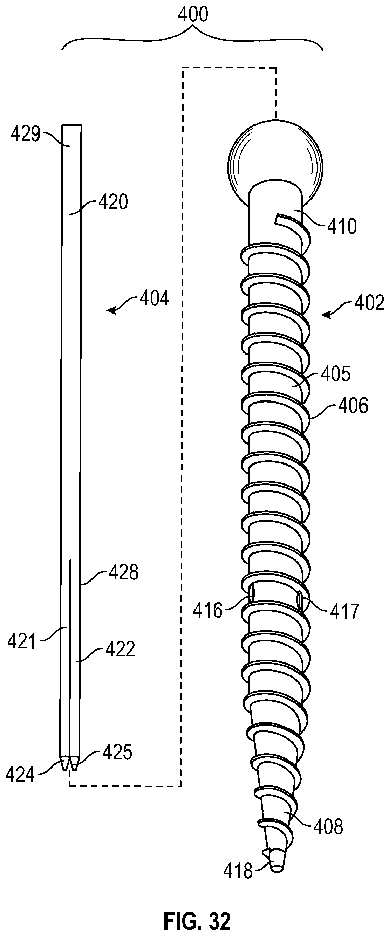

Referring back to FIG. **32****,** in some embodiments the bone anchor **404** defines an elongated proximal portion **420** and a distal portion **428.** The bone anchor **404** further defines the first lateral extension **421** and the second lateral extension **422** defined at the distal portion **428.** In some embodiments, the first lateral extension **421** may include a first pointed end **424** and the second lateral extension **422** may include a second pointed end **425.** In a pre-deployment position shown in FIG. **32****,** the first lateral extension **421** and the second lateral extension **422** may be arranged in a substantially parallel arrangement. In some embodiments, a slight gap, or space, may be defined between the first lateral extension **421** and the second lateral extension **422.** In some embodiments, a thin, breakable connection material, such as a plastic or thin sheet of metal, may be formed between the first lateral extension **421** and the second lateral extension **422** to temporarily maintain the first lateral extension **421** and the second lateral extension **422** in the pre-deployment position. In some embodiments, the bone anchor **404** defines a bendable portion at the base of the first lateral extension **421** and the second lateral extension **422** between the proximal portion **420** and distal portion **428** of the bone anchor **404** that allows the first lateral extension **421** and the second lateral extension **422** of the bone anchor **104** to bend or hinge relative to the proximal portion **420.**

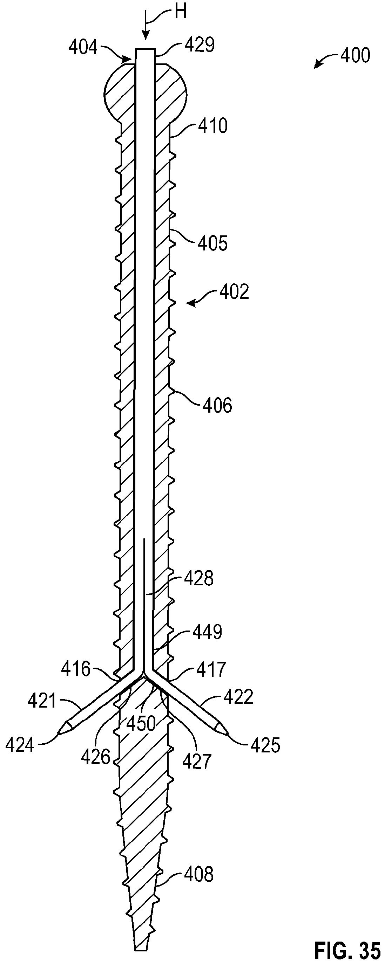

In a post-deployment position, the first lateral extension **421** and the second lateral extension **422** may be received through the axial channel **412** and the first and second lateral channels **426** and **427** respectively and extend outwardly from the first and second lateral openings **416** and **417** respectively. To render the pedicle screw assembly **400** in the post-deployment position, the bone anchor **404** may first be aligned over the pedicle screw **402** with the distal portion **428** oriented towards the proximal opening **414** of the pedicle screw **402.** The bone anchor **404** may then be slidably engaged in a direction **H** through the proximal opening **414** and into the axial channel **412** of the pedicle screw **402** as shown in FIG. **34****.** Subsequently, the bone anchor **404** may continue to be moved in the direction **H** until the distal end **428** of the bone anchor **404** contacts the apex **450** formed at the terminal end of the axial channel **412** opposite the proximal opening **414.** Continual insertion of the bone anchor **404** into the axial channel **412** drives the first pointed end **424** of the first lateral extension **421** and the second pointed end **425** of the second lateral extension **422** against the apex **450,** thereby causing the first lateral extension **421** and the second lateral extension **422** to split away from each other. As such, the first lateral extension **421** passes through the first lateral channel **426** and extends outwardly from the first lateral opening **416,** while the second lateral extension **422** passes through the second lateral channel **427** and extends outwardly from the second lateral opening **417** until the post-deployment position shown in FIG. **35** is achieved (i.e., an inverse "Y" configuration).

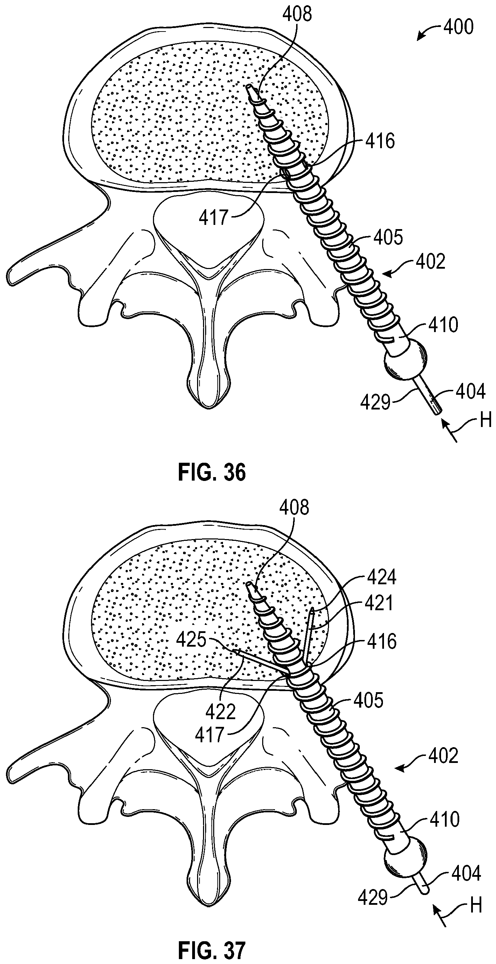

Referring to FIGS. **36-37****,** one method of implanting the pedicle screw assembly **400** will be discussed. As shown in FIG. **36****,** the pedicle screw **402** is first engaged into the bone tissue, such as a pedicle vertebra, such that the proximal portion **410** of the pedicle screw **402** extends from the bone tissue. Once the pedicle screw **402** is implanted into the bone tissue, the bone anchor **404** is inserted into the proximal opening **414** of the pedicle screw **402** until disposed within the axial channel **412** proximate the first and second lateral channels **426** and **427** in a pre-deployment position illustrated in FIG. **34****.**

Once in the pre-deployment position, in some embodiments the user may then further insert the bone anchor **404** until first lateral extension **421** and the second lateral extension of the bone anchor **404** travel through the first and second lateral channels **426** and **427** respectively until extending outwardly from the first and second lateral openings **416** and **417** respectively in a post-deployment position as illustrated in FIG. **35****.** In this post-deployment position, the distal portion **428** of the bone anchor **404** is engaged to the bone tissue as shown in FIG. **37****.**

In some embodiments, the pedicle screw assemblies **100, 200, 300** and **400** are configured to be affixed to the larger vertebrae of the lumbar spine, or the smaller vertebrae of the thoracic or cervical spine.

In some embodiments, the pedicle screw assemblies **100, 200, 300** and **400** may be made from a metal, such as titanium, or a metal-based alloy, such as titanium-based alloy. Alternatively, the pedicle screw assemblies **100, 200, 300** and **400** may be reinforced polymer material. In some embodiments, the material used to manufacture the pedicle screw assemblies **100, 200, 300** and **400** can have a high bioactivity and high flexibility, and a result, can improve ingrowth and mechanical fixation.

In some embodiments, the pedicle screws **102, 202, 302** and **402** may be engaged to a tulip structure (not shown) which is configured to interface with a longitudinal bar or a plate. In some embodiments, the tulip structure can be flexibly coupled to the pedicle screws **102, 202, 302,** and **402** by way of a ball-joint or other type of flexible joint such that the pedicle screw assemblies **100, 200, 300,** and **400** can account for any bending of the individual's spine while still exerting an axial force on the longitudinal bar, thereby stabilizing the spine of the individual.

It should be understood from the foregoing that, while particular embodiments have been illustrated and described, various modifications can be made thereto without departing from the spirit and scope of the invention as will be apparent to those skilled in the art. Such changes and modifications are within the scope and teachings of this invention as defined in the claims appended hereto.

The present invention may also be described or defined in accordance with the following clauses:
1. A pedicle screw assembly, comprising:
   a pedicle screw, comprising
      a screw body, the screw body being elongated and defining a proximal portion and a distal portion,
      an axial channel defined along a longitudinal axis of the screw body, the axial channel in communication with a proximal opening defined along the proximal portion of the screw body,
      a first lateral channel formed along the distal portion of the screw body and in communication with the axial channel, and
      a lateral opening in communication with the first lateral channel; and
   an anchor, comprising
      an anchor body defining an anchor proximal portion and an anchor distal portion, and
      a first lateral extension defined along the anchor distal portion, wherein the axial channel is configured to receive the anchor body and
      the first lateral channel is configured to receive the lateral extension of the bone anchor.
2. The pedicle screw assembly of clause 1, wherein the first lateral channel is oriented towards the proximal portion of the screw body.
3. The pedicle screw assembly of clause 1, wherein the first lateral channel is oriented towards the distal portion of the screw body.
4. The pedicle screw assembly of clause 1, wherein the first lateral extension includes a pointed end configured to drive the first lateral extension within the bone tissue associated with a pedicle vertebra.
5. The pedicle screw assembly of clause 1, further comprising:
   a bendable portion defined along the anchor body of the bone anchor, the first lateral extension being bendable relative to the anchor body at the bendable portion.
6. The pedicle screw assembly of clause 1, wherein the pedicle screw further comprises:
   a second lateral channel in communication the axial channel opposite the first lateral channel.
7. The pedicle screw assembly of clause 6, wherein the axial channel defines a terminal end portion proximate the first and second lateral channels.
8. The pedicle screw assembly of clause 6, wherein an apex is defined between the first and second lateral channels.
9. The pedicle screw assembly of clause 6, the bone anchor further comprises a second lateral extension defined along the anchor distal portion of the bone anchor.
10. The pedicle screw assembly of clause 9, wherein the first lateral extension and the second lateral extension of the bone anchor form an apex such that the first and second lateral extensions are oriented adjacent the anchor proximal portion.
11. The pedicle screw assembly of clause 9, wherein the first lateral extension and the second lateral extension of the bone anchor are oriented in parallel relative to each other.
12. The pedicle screw assembly of clause 1, wherein the pedicle screw further defines a threaded portion configured for engagement within a pedicle vertebra.
13. A pedicle screw assembly, comprising:
   a pedicle screw including an axial channel and at least one lateral channel in communication with a lateral opening and the axial channel;
   a driveshaft configured to be disposed within the axial channel of the pedicle screw; and
   a bone anchor disposed within the at least one lateral channel and configured to be in operative engagement with the driveshaft,
   wherein rotation of the drive shaft causes the bone anchor to extend through the at least one lateral channel.
14. The pedicle screw assembly of clause 13, wherein the driveshaft defines a spline defining a plurality of axially extending peripheral slots around a periphery of the spline.
15. The pedicle screw assembly of clause 14, wherein the bone anchor includes a lateral side defining a gear teeth arrangement.
16. The pedicle screw assembly of clause 15, wherein the gear teeth arrangement is configured to engage the spline of the drive shaft.
17. The pedicle screw assembly of clause 15, wherein a portion of the spline of the driveshaft engages the gear teeth arrangement of the bone anchor such that a rotation of the driveshaft advances the bone anchor outwardly from the lateral opening of the pedicle screw.
18. The pedicle screw assembly of clause 15, wherein rotation of the driveshaft causes the spline to continuously engage the gear teeth arrangement such that the bone anchor is moved incrementally through the lateral channel.
19. A method, comprising:
   providing a pedicle screw including an axial channel, a proximal opening in communication with the axial channel, and a lateral channel in communication with the axial channel;
   providing a bone anchor defining an anchor proximal portion and an anchor distal portion, the bone anchor defining a lateral extension defined at the anchor distal portion;
   orienting the anchor distal portion over the proximal opening of the pedicle screw;
   disposing the bone anchor into the axial channel; and
   moving the lateral extension of the bone anchor through the lateral channel.
20. The method of clause 19, further comprising: applying a force to the anchor proximal portion to drive the lateral extension through the lateral channel.
21. The method of clause 20, further comprising:
   pulling the anchor through the axial channel to apply the force and drive the lateral extension through the lateral channel.
22. The method of clause 20, further comprising:
   pushing the bone anchor through the axial channel towards the lateral extension to apply the force and drive the lateral extension through the lateral channel.
23. The method of clause 19, further comprising:
   applying a rotational force to the anchor proximal portion to drive the lateral extension through the lateral channel.

## Claims

1. A method, comprising:
providing a pedicle screw (100, 200, 300, 400) including an axial channel (112, 212, 312, 412), a proximal opening (114, 314, 414) in communication with the axial channel, a first lateral channel (126, 326, 426) in communication with the axial channel, and a second lateral channel (128, 328, 428) in communication with the axial channel; and
providing a bone anchor (104, 204, 304, 404) defining an anchor proximal portion and an anchor distal portion, the bone anchor defining a first lateral extension (321, 421) defined at the anchor distal portion;
orienting the anchor distal portion over the proximal opening of the pedicle screw;
disposing the bone anchor (104, 204, 304, 404) into the axial channel; and
moving the first lateral extension of the bone anchor through the first lateral channel, wherein the first lateral extension comprises a pointed end configured to penetrate bone tissue.

2. The method of claim 1, further comprising:
applying a force to the anchor proximal portion to drive the first lateral extension (321, 421) through the lateral channel.

3. The method of claim 2, further comprising:
pulling the bone anchor (104, 204, 304, 404) through the axial channel (112, 212, 312, 412) to apply the force and drive the first lateral extension (126, 326, 426) through the first lateral channel.

4. The method of claim 2, further comprising:
pushing the bone anchor (104, 204, 304, 404) through the axial channel (321, 421) towards the first lateral extension to apply the force and drive the first lateral extension through the first lateral channel (112, 212, 312, 412).

5. The method of claim 1, further comprising:
applying a rotational force to the anchor (104, 204, 304, 404) proximal portion to drive the first lateral extension (321, 421) through the first lateral channel (112, 212, 312, 412).
